# EUROPÄISCHE PATENTSCHRIFT

(11) **EP 0 614 912 B1**
(45) Veröffentlichungstag und Bekanntmachung des Hinweises auf die Patenterteilung: **17.05.2000**
(21) Anmeldenummer: 94101703.0
(22) Anmeldetag: 04.02.1994
(51) Int. Cl.: C07K 7/06, C07K 7/08, C07K 14/47, C07K 1/22, G01N 33/92

(54) **Lipoprotein (a)-Peptide und deren Verwendung**
Lipoprotein (a)-peptides and use thereof
Peptides de lipoprotéine (a) et ses applications

(30) Priorität: 09.02.1993 DE 4304638; 31.03.1993 DE 4310516
(43) Veröffentlichungstag der Anmeldung: 14.09.1994
(73) Patentinhaber: Roche Diagnostics GmbH, 68298 Mannheim (DE)
(72) Erfinder: Burns, Geoffrey, Dr., D-80337 München (DE); Engel, Wolf-Dieter, Dr., D-82340 Feldafing (DE); Seidel, Christoph, Dr., D-82362 Weilheim (DE)

(56) Entgegenhaltungen:
- EP-A- 0 327 418
- WO-A-86/04144
- WO-A-92/09893
- US-A- 4 945 040
- JOURNAL OF LIPID RESEARCH Bd. 30, Nr. 1 , Januar 1989 Seiten 23 - 37 H.-C. GUO ET AL. 'Characterization of five mouse monoclonal antibodies to apolipoprotein[a] from human Lp[a]: evidence for weak plasminogen reactivity'

SEQUENZPROTOKOLL

(1) ALLGEMEINE INFORMATION:

(i) ANMELDER:
(A) NAME: Boehringer Mannheim GmbH
(B) STRASSE: Sandhofer Str. 116
(C) ORT: Mannheim
(E) LAND: Deutschland
(F) POSTLEITZAHL: D - 6800

(ii) ANMELDETITEL: Lipoprotein (a)-Peptide und deren Verwendung

(iii) ANZAHL DER SEQUENZEN: 8

(iv) COMPUTER-LESBARE FORM:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.25 (EPA)

(2) INFORMATION ZU SEQ ID NO: 1:

(i) SEQUENZ CHARAKTERISTIKA:
(A) LÄNGE: 12 Aminosäuren
(B) ART: Aminosäure
(C) STRANGFORM: Einzel
(D) TOPOLOGIE: linear

(ii) ART DES MOLEKÜLS: Peptid

(xi) SEQUENZBESCHREIBUNG: SEQ ID NO: 1:

Gln Ala Pro Thr Glu Gln Arg Pro Gly Val Gln Glu
1               5                   10

(2) INFORMATION ZU SEQ ID NO: 2:

(i) SEQUENZ CHARAKTERISTIKA:
(A) LÄNGE: 13 Aminosäuren
(B) ART: Aminosäure
(C) STRANGFORM: Einzel
(D) TOPOLOGIE: linear

(ii) ART DES MOLEKÜLS: Peptid

(xi) SEQUENZBESCHREIBUNG: SEQ ID NO: 2:

Ser Tyr Arg Gly Thr Tyr Ser Thr Thr Val Thr Gly Arg
1               5                   10

(2) INFORMATION ZU SEQ ID NO: 3:

(i) SEQUENZ CHARAKTERISTIKA:
(A) LÄNGE: 12 Aminosäuren
(B) ART: Aminosäure
(C) STRANGFORM: Einzel
(D) TOPOLOGIE: linear

(ii) ART DES MOLEKÜLS: Peptid

(xi) SEQUENZBESCHREIBUNG: SEQ ID NO: 3:

Arg Thr Pro Glu Tyr Tyr Pro Asn Ala Gly Leu Ile
1               5                   10

(2) INFORMATION ZU SEQ ID NO: 4:

(i) SEQUENZ CHARAKTERISTIKA:
(A) LÄNGE: 8 Aminosäuren
(B) ART: Aminosäure
(C) STRANGFORM: Einzel
(D) TOPOLOGIE: linear

(ii) ART DES MOLEKÜLS: Peptid

(xi) SEQUENZBESCHREIBUNG: SEQ ID NO: 4:

Pro Asp Ala Val Ala Ala Pro Tyr
1               5

(2) INFORMATION ZU SEQ ID NO: 5:

(i) SEQUENZ CHARAKTERISTIKA:
(A) LÄNGE: 10 Aminosäuren
(B) ART: Aminosäure
(C) STRANGFORM: Einzel
(D) TOPOLOGIE: linear

(ii) ART DES MOLEKÜLS: Peptid

(xi) SEQUENZBESCHREIBUNG: SEQ ID NO: 5:

Cys Ser Asp Ala Glu Gly Thr Ala Val Ala
1               5                   10

(2) INFORMATION ZU SEQ ID NO: 6:

(i) SEQUENZ CHARAKTERISTIKA:
(A) LÄNGE: 7 Aminosäuren
(B) ART: Aminosäure
(C) STRANGFORM: Einzel
(D) TOPOLOGIE: linear

(ii) ART DES MOLEKÜLS: Peptid

(xi) SEQUENZBESCHREIBUNG: SEQ ID NO: 6:

Thr Pro Val Pro Ser Leu Glu
1               5

(2) INFORMATION ZU SEQ ID NO: 7:

(i) SEQUENZ CHARAKTERISTIKA:
(A) LÄNGE: 11 Aminosäuren
(B) ART: Aminosäure
(C) STRANGFORM: Einzel
(D) TOPOLOGIE: linear

(ii) ART DES MOLEKÜLS: Peptid

(xi) SEQUENZBESCHREIBUNG: SEQ ID NO: 7:

Leu Glu Thr Pro Thr Val Val Pro Val Pro Ser
1               5                   10

(2) INFORMATION ZU SEQ ID NO: 8:

(i) SEQUENZ CHARAKTERISTIKA:
(A) LÄNGE: 8 Aminosäuren
(B) ART: Aminosäure
(C) STRANGFORM: Einzel
(D) TOPOLOGIE: linear

(ii) ART DES MOLEKÜLS: Peptid

(xi) SEQUENZBESCHREIBUNG: SEQ ID NO: 8:

Thr Phe Ile Pro Gly Thr Asn Lys
1               5

## Beschreibung

Gegenstand der Erfindung sind Peptide, die eine Teilsequenz von Lipoprotein (a) aufweisen, sowie deren Verwendung zur affinitätschromatographischen Aufreinigung von Antikörpern, als Immunogen zur Herstellung von Antikörpern sowie als Standard in einem immunologischen Test oder als kompetitierendes Hapten in einem Agglutinationstest.

Eine erhöhte Konzentration von Lipoprotein (a) (Lp(a)) stellt einen von der LDL-Konzentration unabhängigen Risikofaktor für einen Herzinfarkt oder Schlaganfall dar (H.-C. Guo et al., Journal of Lipid Research 30 (1989), 23 - 37). Die Bedeutung dieses Risikofaktors für die Diagnostik liegt insbesondere darin, daß die Lp(a)-Konzentration nicht durch Ernährungsgewohnheiten oder eine Therapie mit Hydroxymethylglutaryl-CoA-Reduktase-Inhibitoren beeinflußt wird, so daß über die Bestimmung der Lp(a)-Konzentration ein wichtiger Hinweis auf eine genetische Disposition für einen Herzinfarkt oder Schlaganfall erhalten werden kann (C. Labeur et al., Clinical Chemistry 35 (1989), 1380 - 1384). Der spezifische diagnostische Nachweis von Lp(a) wird jedoch dadurch erschwert, daß Lp(a) einerseits in seinem hohen Gehalt an Cholesterinestern und dem Bestandteil Apoprotein B100 dem LDL sehr ähnlich ist, andererseits das Lp(a) von LDL unterscheidende Apoprotein (a) eine sehr hohe Homologie zum Plasminogen aufweist (H.-C. Guo et al., Journal of Lipid Research 30 (1989), 23 - 37). Die durch Immunisierung mit Lp(a) erhaltenen Antiseren reagieren daher nicht nur mit Lp(a), sondern in erheblichem Umfang auch mit LDL und/oder Plasminogen.

Um dennoch möglichst spezifisch Lp(a) nachweisen zu können, wurden Antiseren gegen Lp(a) durch eine aufwendige immunadsorptive Reinigung an LDL, Lp(a), Epitope von Apoliprotein A (WO-A-92/09893) oder Apolipoprotein (a) aufgereinigt (Kraft et al., Arteriosclerosis 8 (1988), 212 - 216). Daneben wurde versucht, einen spezifischen Lp(a)-Nachweis über eine spezielle ELISA-Testführung zu erreichen (Fless et al., Journal of Lipid Research 30 (1989), 651 - 662). Hierbei wird z.B. ein Antikörper gegen das Apolipoprotein (a) zur Immobilisierung von nachzuweisendem Lp(a) aus der Probe verwendet und ein Antikörper gegen das Apolipoprotein B100 zum Nachweis von immobilisiertem Lp(a) eingesetzt. Diese Verfahren sind jedoch sehr aufwendig. Zudem ist bei den physiologischen Konzentrationen von Lp(a) (bis zu über 1 mg/ml) für eine Bestimmung gemäß dem ELISA-Prinzip eine hohe (100- bis 1000fache) Probenverdünnung erforderlich, die sehr störanfällig ist.

Aufgabe der Erfindung war es daher, eine einfachere Aufreinigung von Antikörpern gegen Lp(a) sowie ein zuverlässigereres Nachweisverfahren zur spezifischen Bestimmung von Lp(a) zur Verfügung zu stellen.

Diese Aufgabe wird gelöst durch ein Peptid, das eine der in SEQ ID NO: 1-8 gezeigten Sequenzen enthält und im CD-Spektrum eine negative Bande zwischen 190 und 200 nm zeigt. In dem Fachmann geläufiger Weise kann davon ausgegangen werden, daß Peptide, die nur einen Teil der in SEQ ID NO 1-8 gezeigten sequenzen enthalten, in analoger Weise wirken und verwendet werden können, sofern sie mindestens vier Aminosäuren enthalten.

Wesentlich für die Eignung der erfindungsgemäßen Peptidantigene ist, daß sie nicht in einer definierten gefalteten Struktur, sondern linear vorliegen. Eine solche lineare Struktur wird auch als random-coil-Struktur bezeichnet. Ob für ein Oligopeptid eine solche Struktur vorliegt, läßt sich durch CD-Spektroskopie überprüfen. Ein Peptid mit randomcoil-Struktur besitzt zwischen 190 und 200 nm im CD-Spektrum eine negative Bande (vgl. Ann. Rev. Biophys. Chem. 17 (1988), 145 - 166).

Die erfindungsgemäßen Peptide werden in bekannter Weise synthetisch hergestellt, vorzugsweise mittels Fluorenyloxycarbonyl-Festphasen-Synthese.

Es hat sich überraschenderweise gezeigt, daß mit Hilfe dieser Peptide in einem einzigen affinitätschromatographischen Schritt Antikörper gegen Lp(a) erhalten werden können, welche nur eine geringe Reaktivität mit LDL und/oder Plasminogen aufweisen. Unter einer geringen Reaktivität ist dabei zu verstehen, daß die Kreuzreaktivität mit LDL und/oder Plasminogen maximal 1,0 %, bezogen auf die Reaktivität mit Lp(a), beträgt.

Ein weiterer Gegenstand der Erfindung ist daher (a) die Verwendung eines erfindungsgemäßen Peptids zur affinitätschromatographischen Aufreinigung von Antikörpern gegen Lp(a), wobei man das Peptid an ein Trägermaterial bindet, an dem dann der Antikörper in an sich bekannter Weise affinitätschromatographisch aufgereinigt werden kann beziehungsweise (b) ein Verfahren zur affinitätschromatographischen Aufreinigung von Antikörpern gegen Lp(a), wobei ein erfindungsgemäßes Peptid an ein Trägermaterial gebunden ist, an dem der Antikörper dann in an sich bekannter Weise affinitätschromatisch aufgereinigt wird.

Als Trägermaterial eignen sich alle üblicherweise in der Affinitätschromatographie eingesetzten Trägermaterialien, vorzugsweise wird Sepharose-AH (Pharmacia LKB) oder Affi-Gel 10 (BioRad) verwendet. Die Bindung des Peptids an Sepharose-AH erfolgt dabei in bekannter Weise durch Aktivierung des Peptids mit Maleinimidobenzoyl-N-hydroxysuccinimidester (MBS) oder Glutardialdehyd. Die Bindung an Affi-Gel 10 erfordert keine Aktivierung des Peptids und erfolgt nach Angaben des Herstellers. Die Aufreinigung des Antiserums über die so erhaltene Affinitätschromatographiematrix erfolgt in an sich bekannter Weise durch Auftragen des Antiserums, Waschen mit einem Puffer hoher Ionenstärke und anschließender Elution mit einem Puffer geringer Ionenstärke und einem pH-Wert unter 5. Vorzugsweise wird mit PBS (nach Dulbecco und Vogt, J. Exp. Med. 99 (1954), 167 - 182) / 0,5 mol/l Natriumchlorid gewaschen und mit 0,2 mol/l Glycin HCl pH 2,6 eluiert. Die so erhaltenen Antikörper zeigen nur eine geringe Reaktivität mit LDL und Plasminogen.

Weiterhin eignen sich die erfindungsgemäßen Peptide als Immunogen oder Hapten zur Herstellung von Antikörpern gegen Lp(a), die nur eine geringe Reaktivität mit LDL und/oder Plasminogen aufweisen.

Ein weiterer Gegenstand der Erfindung ist daher (a) die Verwendung eines erfindungsgemäßen Peptids als Immunogen zur Herstellung von Antikörpern gegen Lp(a), die nur eine geringe Reaktivität mit LDL und/oder Plasminogen aufweisen beziehungsweise (b) ein Verfahren zur Herstellung von Antikörpern gegen Lp(a), die nur eine geringe Reaktivität mit LDL und/oder Plasmeinogen aufweisen, wobei als Immunogen ein erfindungsgemäßes Peptid eingesetzt wird. Die Peptide können dabei als solche oder gebunden an ein Trägermolekül zur Immunisierung eingesetzt werden. Die Immunisierung erfolgt in an sich bekannter Weise in den hierfür üblicherweise verwendeten Tieren. Vorzugsweise werden Kaninchen, Ziegen, Ratten, Schafe oder, zur Herstellung monoklonaler Antikörper, Mäuse verwendet. Das erhaltene Antiserum kann vorzugsweise wie oben beschrieben affinititätschromatographisch aufgereinigt werden. Zur Herstellung monoklonaler Antikörper werden die Milzzellen der immunisierten Tiere nach bekannten Verfahren immortalisiert und diejenigen immortalisierten Zellen, deren Kulturüberstand einen Antikörper gegen Lp(a) aufweist, kloniert. Ob ein Kulturüberstand einen Antikörper gegen Lp(a) aufweist, wird in üblicher Weise über einen ELISA-Test bestimmt.

Ein weiterer Vorteil der erfindungsgemäßen Peptide liegt darin, daß sie in großen Mengen in einheitlicher Zusammensetzung erhalten werden können.

Sie eignen sich daher besser als Standard für quantitative Bestimmungen von Lp(a) als das aus natürlichen Quellen isolierte, sehr uneinheitliche Material.

Ein weiterer Gegenstand der Erfindung ist daher die Verwendung eines erfindungsgemäßen Peptids als Standard in einem immunologischen Test zur quantitativen Bestimmung von Lp(a). Die immunologische Bestimmung von Lp(a) kann dabei nach allen bekannten Verfahren erfolgen. In bestimmten Fällen, wie z.B. bei nicht kompetitiven Testsystemen, ist es erforderlich, mehrere erfindungsgemäße Peptide gleicher oder unterschiedlicher Sequenz an ein Trägermolekül zu binden.

Ein weiterer Gegenstand der Erfindung ist auch die Verwendung eines erfindungsgemäßen Peptids als Hapten in einem kompetitiven immunologischen Test. Vorzugsweise werden dazu die erfindungsgemäßen Peptide biotinyliert, an eine mit Streptavidin beschichtete feste Phase gebunden und der kompetitive Test in üblicher Weise nach dem ELISA-Prinzip durchgeführt. Das erfindungsgemäße Peptid kann jedoch bei einem kompetitiven Test nach dem ELISA-Prinzip auch an den Marker (Enzym, Fluoreszenzmarker) gebunden werden.

Bei einem Immunoassay nach dem ELISA-Prinzip ist aller-dings bei den physiologischen Konzentrationen von Lp(a) eine hohe und damit zeitaufwendige und fehleranfällige Probenvorverdünnung erforderlich. Die hier vom Konzentrationsbereich her besser geeignetere turbidimetrische Bestimmungsmethode (TINIA = turbidimetrischer Inhibitions-Immunoassay) wurde bislang dadurch erschwert, daß Lp(a) sowohl mit polyklonalen als auch mit monoklonalen Antikörpern nur unter bestimmten Bedingungen ausreichende Trübungssignale bewirkt. Eine solche ausreichende Trübung kann jedoch erreicht werden, wenn einzelne oder mehrere erfindungsgemäße Peptide an einen Träger gekoppelt werden und der erhaltene Komplex als Hapten im Agglutinationstest eingesetzt wird. Durch den zu bestimmenden Analyten wird diese Trübung dann in einem zur Menge des Analyten proportionalen Ausmaß reduziert. Vorzugsweise werden 30 bis 40 Peptidmoleküle je Trägermolekül gekoppelt. Die Bindung des erfindungsgemäßen Peptids an den Träger kann dabei direkt über kovalente Bindungen erfolgen oder aber indirekt, z.B. durch Biotinylierung des Peptids und eine Streptavidin-Beschichtung des Trägermaterials. Als Trägermolekül werden dabei vorzugsweise Proteine wie Immunglobuline, Albumin, β-Galactosidase, Polymere wie Aminodextrane oder Polylysine oder Partikel wie Latex oder Gold, jeweils allein oder in Kombination miteinander, verwendet. Die Kopplung an das Trägermolekül erfolgt in bekannter Weise, z.B. mit Reagenzien wie Glutardialdehyd, Ethyldimethylaminopropylcarbodiimid, Maleinimidohexansäure-N-hydroxysuccinimidester oder anderen bekannten homo- und heterobifunktionellen Linkern.

Ein weiterer Gegenstand der Erfindung ist daher die Verwendung eines erfindungsgemäßen Peptids als Lp(a)-Hapten in einem Agglutinationstest zur Bestimmung von Lp(a), wobei mindestens ein erfindungsgemäßes Peptid an einen Träger gebunden wird, so daß sich bei Inkubation des dadurch gebildeten Komplexes mit einem Antikörper, der das entsprechende Lp(a)-Hapten erkennt, ein Agglutinat bildet, dessen Ausbildung in Gegenwart von freiem Lp(a) aus der Probe vermindert wird.

Ein weiterer Gegenstand der Erfindung ist schließlich ein Verfahren zur immunologischen Bestimmung von Lp(a) über einen kompetitiven Agglutinationstest durch Inkubation eines trägergebundenen Lp(a)-Haptens mit einem Antikörper gegen dieses Hapten und der zu analysierenden Probe und Messung des dabei auftretenden Meßsignals in Gegenwart und Abwesenheit der zu analysierenden Probe, wobei das trägergebundene Lp(a)-Hapten mindestens ein erfindungsgemäßes Peptid enthält.

Der Agglutinationstest wird dabei in an sich bekannter Weise durchgeführt. Dazu wird z.B. zunächst das trägergebundene Lp(a)-Hapten mit einem Antikörper inkubiert, der dieses Hapten erkennt und daher einen Komplex mit dem trägergebundenen Lp(a)-Hapten bildet, der zu einem bestimmten Meßsignal, in der Regel einer bestimmten Trübung der Reagenzlösung, führt. Nach Zugabe der zu analysierenden Probe kompetiert freies Lp(a) aus dieser Probe mit dem trägergebundenen Lp(a)-Hapten um die Bindung an den Antikörper und verringert somit die Aggregation des trägergebundenen Lp(a)-Haptens durch den Antikörper und damit die Trübung. Die gemessene Trübungsabnahme wird verglichen mit der Trübungsabnahme, die durch Zugabe von bekannten Mengen eines Lp(a)-Standards erhalten wird, und aus dem Vergleich die Menge an Lp(a) in der zu analysierenden Probe ermittelt. Als Standard wird dabei vorzugsweise ein erfindungsgemäßes Peptid verwendet. Daneben kann der Agglutinationstest aber auch nach anderen dem Fachmann geläufigen Verfahren einer kompetitiven Testführung durchgeführt werden. Z.B. kann das trägergebundene Lp(a)-Hapten auch gleichzeitig mit der Probe und dem Antikörper inkubiert werden, wobei das freie Lp(a) aus der Probe mit dem trägergebundenen Hapten um die Bindung an den Antikörper konkurriert, so daß die beobachtete Agglutination umgekehrt proportional zu der Menge an freiem Lp(a) in der Probe ist. Eine weitere Möglichkeit ist, zunächst den Antikörper mit der Probe zu inkubieren, wodurch eine der Menge an freiem Lp(a) in der Probe entsprechende Menge an Antikörper gebunden wird. Nach Inkubation mit dem trägergebundenen Lp(a)-Hapten erfolgt eine Agglutination, die der Menge an freiem Lp(a) in der Probe direkt proportional ist.

Die Bestimmung der Lp(a)-Konzentration erfolgt dabei entweder auf molarer Basis (bei Verwendung eines Peptides, das nur einmal in Lp(a) vorkommt, z.B. das Peptid der in SEQ ID NO 8 gezeigten Sequenz) oder auf Massenbasis (die bei Verwendung eines Peptides, das mehrmals per Lp(a)-Molekül vorkommt, z.B. die Peptide mit der in SEQ ID NO 1-7 gezeigten Sequenz).

Ein weiterer Gegenstand der Erfindung ist ein Verfahren zur immunologischen Bestimmung von Lp(a) durch Inkubation eines Marker-gebundenen Lp(a)-Haptens mit einem Antikörper gegen dieses Hapten und der zu analysierenden Probe und Messung des dabei auftretenden Meßsignals in Gegenwart und Abwesenheit der zu analysierenden Probe, dadurch gekennzeichnet, daß das Marker-gebundene Lp(a)-Hapten mindestens ein erfindungsgemäßes Peptid enthält. Als Marker können dabei alle üblicherweise verwendeten Marker wie Enzyme, Enzymfragmente, Chemilumineszenz- oder Fluoreszenzfarbstoffe und radioaktive Isotope verwendet werden. Vorzugsweise erfolgt die Bestimmung nach dem FPIA-, EMIT- oder CEDIA-Prinzip.

Beim Fluoreszenz-Polarisations-Immunoassay (FPIA) wird das Hapten mit einer fluoreszierenden Substanz markiert. Diese Moleküle absorbieren Lichtenergie und geben sie in einem Zeitraum von etwa 10⁻⁸ sec als Licht längerer Wellenlänge wieder ab. Wird der Fluorophor durch polarisiertes Licht angeregt, so hängt der Polarisationsgrad des emittierten Lichts von der Rotationsgeschwindigkeit des Tracers (Analyt-Fluorophor-Konjugat) ab. Die Bindung des Tracers an einen Antikörper behindert die Rotation des Fluorophors. Der freie Tracer rotiert schneller und depolarisiert das anregende Licht mehr als der größere, trägere Antikörper-Tracer-Komplex. Je mehr der Analyt in der Probe vorhanden ist, desto weniger Antikörper-Tracer-Komplexe entstehen und desto weniger Fluoreszenzpolarisation ist meßbar (W. Dandliker et al., Journal of Exp. Med. 122 (1965), 1029).

Bei der Enzyme Multiplied Immunoassay Technique (EMIT) wird das nachzuweisende Hapten so kovalent mit dem Markerenzym gekoppelt, daß die Enzymaktivität erhalten bleibt. Nach Bindung eines Antikörpers an den Haptenanteil wird die Substratbindung an das Enzym sterisch jedoch behindert, so daß keine enzymatische Umsetzung des Substrats erfolgen kann. Wie beim CEDIA-Prinzip verdrängt dann auch hier das Antigen aus der zu bestimmenden Probelösung den Antikörper vom enzymgebundenen Hapten und ermöglicht so eine enzymatische Aktivität, die proportional zur Konzentration des zu analysierenden Antigens in der Probelösung ist (Gunzer et al., Kontakte III, 1980, 3 - 11 und K. Rubenstein, Biochemical and Biophysical Research Communications 47 (1972), 846 - 851).

Beim CEDIA-Prinzip bewirkt das Antigen aus der zu analysierenden Probe die Assoziation von allein jeweils inaktivem Enzymakzeptor und Enzymdonor zu einem aktiven Enzym, dessen Aktivität somit proportional zur Menge an Antigen in der zu analysierenden Probe ist (Henderson et al., Clinical Chemistry 32 (1986), 1637 - 1641). Zum Nachweis werden hierbei bestimmte Enzyme wie z.B. die β-Galactosidase verwendet, die in zwei jeweils enzymatisch inaktiven Bestandteilen, nämlich einem großen Polypeptid (Enzymakzeptor) und einem kleinen Polypeptid (Enzymdonor) vorliegen, wobei diese Bestandteile spontan zu einem enzymatisch aktiven Protein assoziieren. An den Enzymdonor wird das als Analyt nachzuweisende Hapten derart gebunden, daß die Assoziation des Enzymdonors mit dem Enzymakzeptor zum aktiven Enzym durch diese Bindung nicht verhindert wird. Diese Assoziation wird aber dann gehemmt, wenn an den Antigen-Enzymdonor-Komplex ein Antikörper gegen das Antigen bindet. In einer Reagenzlösung, in der Enzymakzeptor, Antigen-Enzymdonor-Komplex und der entsprechende Antikörper vorliegen, kann daher kein aktives Enzym gebildet werden und es wird keine enzymatische Aktivität gemessen. Nach Zugabe der Probelösung verdrängt nun das Antigen aus dieser Probelösung den Antikörper aus der Bindung an den Antigen-Enzymdonor-Komplex und ermöglicht so die Ausbildung des aktiven Enzyms.

Die Antikörper, die mit dem erfindungsgemäßen Verfahren zur affinitätschromatischen Aufreinigung von Antikörpern gegen Lp(a), wobei ein erfindungsgemäßes Peptid an ein Trägermaterial gebunden ist, an dem der Antikörper dann in an sich bekannter Weise affinitätschromatisch aufgereinigt wird oder mit dem Verfahren zur Herstellung von Antikörpern gegen Lp(a), die nur eine geringe Reaktivität mit LDL und/oder Plasminogen aufweisen, wobei als Immunogen ein erfindungsgemäßes Peptid eingesetzt wird, hergestellt wurden, sind bei der immunologischen Bestimmung von Lp(a) besonders geeignet. Ein weiterer Gegenstand der Erfindung ist daher die Verwendung dieser erfindungsgemäß erhältlichen Antikörper bei der immunologischen Bestimmung von Lp(a).

Die Erfindung wird durch die folgenden Beispiele zusammen mit den Sequenzprotokollen 1-8, welche die Sequenz der erfindungsgemäßen Peptide angeben, näher erläutert.

### Beispiel 1

### Peptidsynthese

Das Peptid mit der um ein C-terminales Cys verlängerten Sequenz SEQ ID NO 1 wird mittels Fluorenyloxycarbonyl(Fmoc)-Festphasensynthese hergestellt. Die Reaktionen werden an einem Labortec SP 640 Peptidsynthesizer (Labortec, Schweiz) durchgeführt. Die Synthese erfolgt dabei an 5 g Wang-Harz (Polystyrol/1 % Divinylbenzol) mit einer Beladung an Peptid von 0,5 mmol/g (entsprechend JACS 95 (1973), 1328). Die Kupplung erfolgt durch Inkubation des Harzes mit dem entsprechenden Fmoc-Aminosäurederivat (1 Äquivalent) sowie 1,2 Äquivalenten Dicyclohexylcarbodiimid und 1,1 Äquivalenten N-Hydroxybenzotriazol für 90 Min. bei Raumtemperatur in Dimethylformamid als Reaktionsmedium. Von den Fmoc-Aminosäurederivaten werden je 4 Äquivalente bezogen auf 1 Mol Ankergruppierung in folgender Reihenfolge verwendet: Glutamin (mit Trityl.-Schutzgruppe), Alanin, Prolin, Threonin (mit tert. Butyl-Schutzgruppe), Glutaminsäure (mit tert. Butylester-Schutzgruppe), Glutamin (mit Trityl-Schutzgruppe), Arginin (mit Pentamethylchroman-Schutzgruppe), Prolin, Glycin, Valin, Glutamin (mit Trityl-Schutzgruppe), Glutaminsäure (mit tert. Butylester-Schutzgruppe) und Cystein (mit Trityl-Schutzgruppe). Der Kupplungserfolg wird mittels Kaisertest (Anal. Biochem. 34 (1970), 595) geprüft. Nach der Kupplung wird die Fmoc-Gruppe durch Inkubation mit 20 % Piperidin in Dimethylformamid für 10-20 Min. bei Raumtemperatur abgespalten. Die Harzbeladung wird mittels UV-Absorption der freigesetzten Fulven-Gruppe nach jeder Piperidinspaltung ermittelt. Nach vollständiger Synthese des Peptids beträgt der Beladungsgrad noch 0,43 mmol/g.

Die Freisetzung des Peptids vom Harz erfolgt durch Inkubation mit 200 ml Trifluoressigsäure, 20 ml Ethandithiol, 20 ml m-Kresol und 10 ml Wasser für 30 Min. bei Raumtemperatur. Die Reaktionslösung wird unter Sauerstoffausschluß anschließend mehrmals mit Toluol eingeengt und dann das Peptid mit peroxidfreiem Diethylether ausgefällt.

Zur Aufreinigung wird das erhaltene Rohmaterial unter N₂-Atmosphäre über eine Sephadex-G10-Säule gereinigt. Es werden nach Lyophilisieren 2,4 g Material einer Reinheit von 54 % gemäß RP-HPLC erhalten.

Zur weiteren Aufreinigung werden 400 mg dieses Peptids über eine präparative RP-HPLC-Säule (40 mm x 250 mm C18-Material, 5 µm, 300 Å) mit einem Wasser/Trifluoressigsäure zu Acetontril/Trifluoressigsäure-Gradienten gereinigt (Puffer A: 0,1 % Trifluoressigsäure in Wasser, Puffer B: 0,1 % Trifluoressigsäure in Wasser/Acetonitril 60:40 von 0 % B nach 100 % B in 60 min.). Nach Lyophilisation werden 97 mg weißes Material mit einer gemäß HPLC 97,2%igen Reinheit erhalten. Die Identität des erhaltenen Peptids wird mittels FAB-MS überprüft.

### Beispiel 2

### Biotinylierung des Peptids

Zur Biotinylierung eines Moläquivalents des gemäß Beispiel 1 erhaltenen Peptidantigens wird das Peptid in einer Konzentration von 5 mg/ml in Argon-gesättigtem Kaliumphosphatpuffer (0,1 mol/l pH 8,0) gelöst, mit 3 Äquivalenten D-Biotinyl-ε-aminocapronsäure-N-hydroxysuccinimidester (1 µmol gelöst in 5 µl Argon-gesättigtem Dimethylformamid) versetzt und 2 h bei Raumtemperatur unter Rühren und Argonatmosphäre inkubiert. Sobald gemäß Kontrolle über analytische RP-HPLC die Ausgangsprodukte auf weniger als 5 % abgenommen haben, wird der Reaktionsansatz direkt auf eine präparative RP-HPLC-Säule gegeben und das Produkt über einen 0,1 % Trifluoressigsäure/Wasser zu 0,1 % Trifluoressigsäure/Acetonitril-Gradienten (0 % bis 100 % Acetonitril in 90 Min.) aufgereinigt. Das Produkt wird eingeengt und lyophilisiert, die Ausbeute beträgt zwischen 40 und 90 %. Die Reinheit des erhaltenen Materials wird über HPLC, Kapillarelektrophorese und DC bestimmt, die Identität über FAB-MS und DC mit spezifischen Anfärbereagenzien (p-Dimethylaminozimtaldehyd für den Gehalt an Biotin) sowie Gehaltsbestimmung über Mikroanalyse.

### Beispiel 3

### Affinitätschromatographische Aufreinigung von Antiseren gegen Lp(a)

Das Immunabsorbens wird wie von Chersi et al. (J. Immunol. Meth. 122 (1989), 285 - 289) beschrieben hergestellt. Hierzu wird Sepharose-AH (3 ml gepacktes Gel in 6 ml PBS) mit Maleinimidobenzoyl-N-hydroxysuccinimidester (MBS, 5 mg/ml in Dimethylformamid) für 2 h bei Raumtemperatur unter vorsichtigem Mischen behandelt. Überschüssiger MBS wird anschließend durch Zentrifugation bei 2000 g für 15 Min. bei 4°C entfernt. Um das Gelmaterial zu waschen, wird es in PBS resuspendiert und nochmals abzentrifugiert. Diese Waschzyklen werden noch mehrfach wiederholt. Nach dem letzten Zentrifugationsschritt werden 2 mg einer äquimolaren Mischung der Peptide mit den in SEQ ID NO 1-8 gezeigten Sequenzen in 4 ml PBS zum Gel hinzugegeben, vorsichtig gemischt und für 60 Min. bei Raumtemperatur inkubiert. Die Suspension wird daraufhin ad 10 mmol/l Mercaptoethanol aufgestockt, nochmals 30 Min. inkubiert, in eine kleine Säule gefüllt und mit PBS/10 mmol/l Mercaptoethanol gewaschen. Vor der Aufreinigung der Antikörper wird das Gelmaterial dem folgenden Waschzyklus unterworfen. Gewaschen wird mit mindestens 3 Säulenvolumen PBS, 0,5 mmol/l NaCl/0,05 % Tween 20, 30 mmol/l NaCl, 0,2 mol/l Glycin, pH 2,6 und 30 mmol/l NaCl. Anschließend wird das Gelmaterial mit PBS, pH 7,0 equilibriert und das den Antikörper enthaltende aufzureinigende Serum auf die Säule aufgetragen. Nach Waschschritten mit PBS, 0,5 mol/l NaCl/0,05 % Tween 20® und 30 mmol/l NaCl wird der gebundene Antikörper mit 0,2 mol/l Glycin, pH 2,6 eluiert und direkt anschließend bei 4°C gegen PBS dialysiert.

### Beispiel 4

### Bestimmung der Spezifität der immunadsorptiv gereinigten Antikörper

Die Spezifität der gemäß Beispiel 3 erhaltenen Antikörper wird über einen Sandwich-Assay bestimmt. Dazu werden die folgenden Reagenzien verwendet:

### Reagenz 1:

Gemäß Beispiel 1 hergestelltes biotinyliertes Peptid (0,2 µg/ml)
40 mmol/l Phosphatpuffer pH 7,0,
0,9 % Natriumchlorid
10 % BSA.

### Reagenz 2:

20 mU/ml Peroxidase-markierter Fab-Fragmente gegen Schafimmunglobulin G (Boehringer Mannheim GmbH, Kat.-Nr. 1301 977)
40 mmol/l Phosphatpuffer pH 7,0
0,5 % Tween 20
0,2 % BSA
0,2 % Rinderimmunglobulin G (Sigma, Kat.-Nr. I 5506)

Zur Versuchsdurchführung wird 1 ml Reagenz 1 und 10 µl der zu testenden Antikörperlösung 1:100 verdünnt in Streptavidin-beschichtete Polystyrolröhrchen (hergestellt gemäß Beispiel 1 von EP-A 344 578) pipettiert und 1 h bei Raumtemperatur inkubiert. Die Röhrchen werden dann 3 x mit normalem Leitungswasser gewaschen und anschließend mit 1 ml Reagenz 2 für 1 h bei Raumtemperatur inkubiert und dann nochmals 3 x mit Leitungswasser gewaschen. Nach Zugabe von 1 ml ABTS® in 100 mmol/l Phosphat-Citratpuffer pH 4,4, der 3,2 mmol/l Natriumperborat enthält (Boehringer Mannheim GmbH, Kat.-Nr. 746407) und Inkubation für 60 Min. bei Raumtemperatur wird dann die Bindung der markierten Fab-Fragmente durch Messung der Extinktion bei 420 nm bestimmt. Die nachfolgende Tabelle zeigt die Reaktivität von 8 untersuchten Antiseren mit den Peptiden der in SEQ ID NO 1-8 gezeigten Sequenz. Als positiv wird dabei ein Signal gewertet, das einen um 3 Standardabweichungen höheren Wert als der Mittelwert von 10 negativen Kontrollseren aufweist.

**Tabelle 1**

| Antiserum | SEQ ID NO: | | | | | | | |
|---|---|---|---|---|---|---|---|---|
| | 1 | 2 | 3 | 4 | 5 | 6 | 7 | 8 |
| a | + | + | + | + | + | + | + | - |
| b | + | - | - | - | + | - | + | - |
| c | - | - | + | - | - | - | - | - |
| d | - | - | - | + | - | - | - | - |
| e | - | - | - | - | + | - | - | - |
| f | + | + | + | - | - | - | - | - |
| g | - | - | - | - | - | - | + | + |
| h | + | - | - | + | - | - | + | + |

### Beispiel 5

### Kreuzreaktivität der immunadsorptiv gereinigten Antikörper

Zur Bestimmung der Kreuzreaktivität des Antiserums a (siehe Beispiel 4, Tabelle 1) wird der in Beispiel 4 beschriebene Sandwich-Immunoassay in Gegenwart verschiedener Konzentrationen an Lp(a), LDL bzw. Plasminogen (Konzentrationen siehe Tabelle 2) durchgeführt. Hierbei konkurrieren diese zugesetzten freien Antigene mit den immobilisierten biotinylierten Peptiden um die Bindung des Antiserums, so daß die Bindung des Antiserums an die feste Phase und damit die Signalstärke in einem Ausmaß abnimmt, das der Reaktivität der zugesetzten freien Antigene mit dem Antiserum entspricht. Je mehr freies Antigen für eine Reduzierung des erhaltenen Signals benötigt wird, desto geringer ist also die Reaktivität des Antiserums mit diesem Antigen. Auf diese Weise wird das Antiserum a vor und nach der affinitätschromatographischen Aufreinigung gemäß Beispiel 3 untersucht. Wie die nachfolgende Tabelle 2 zeigt, kann die Kreuzreaktivität des Antiserums a gegenüber LDL und Plasminogen durch die affinitätschromatographische Aufreinigung deutlich reduziert werden.

**Tabelle 2**

| Substanz/Konzentration (µmol/l) | Signal | |
|---|---|---|
| | Vor Aufreinigung | Nach Aufreinigung |
| **Lp(a)** | | |
| 0 | 287 | 241 |
| 0,5 | 270 | 228 |
| 1,0 | 237 | 174 |
| 2,0 | 77 | 40 |
| 3,0 | 12 | 8 |

| **LDL** | | |
|---|---|---|
| 0 | 287 | 241 |
| 2,0 | 268 | 240 |
| 5,0 | 232 | 241 |
| 10,0 | 140 | 240 |
| 20,0 | 66 | 239 |
| 40,0 | 7 | 242 |
| 100,0 | 0 | 235 |
| 200,0 | 0 | 218 |
| 500,0 | 0 | 104 |
| 1000,0 | 0 | 29 |
| 2000,0 | 0 | 0 |

| **Plasminogen** | | |
|---|---|---|
| 0 | 287 | 241 |
| 2,0 | 274 | 240 |
| 5,0 | 260 | 242 |
| 10,0 | 226 | 243 |
| 20,0 | 149 | 242 |
| 40,0 | 62 | 241 |
| 100,0 | 1 | 239 |
| 200,0 | 0 | 230 |
| 500,0 | 0 | 172 |
| 1000,0 | 0 | 53 |
| 2000,0 | 0 | 7 |

### Beispiel 6

### Agglutinationstest zur immunologischen Bestimmung von Lp(a)

Lp(a) wird in einem homogenen Immunoassay bestimmt. Zum Nachweis werden die Reagenzien mit folgender Zusammensetzung verwendet:

### Reagenz 1:

monoklonaler Antikörper gegen Peptid 1 (75 µg/ml, Boehringer Mannheim GmbH, Kat.-Nr. 1411 012)
Polystreptavidin (20 µg/ml)
20 mmol/l MES, pH 6,0
225 mmol/l NaCl
4,5 % PEG 6000
0,75 % Brij 35
0,1 % NaN₃

### Reagenz 2:

20 mmol/l MES, pH 6,0
150 mmol/l NaCl
6,0 % PEG 6000
0,5 % Brij 35
0,1 % NaN₃
biotinyliertes Peptid 1 (2 µg/ml, siehe Beispiel 2)

Die Messungen werden an einem Hitachi 704 bei 37°C durchgeführt. In einer Küvette werden 350 µl Reagenz 1 und 10 µl Probe 5 Min. inkubiert. Anschließend wird 70 µl Reagenz 2 zupipettiert und für 5 Min. inkubiert und die Änderung der Adsorption bei 340 nm bestimmt. Die gemessene Trübung wird verglichen mit der Trübung, die durch Zugabe von bekannten Mengen eines Lp(a)-Standards erhalten wird, und aus dem Vergleich die Menge an Lp(a) in der zu analysierenden Probe ermittelt.

## Patentansprüche

1. Verwendung eines Peptids der Sequenzen SEQ ID NO 1-8 QAPTEQRPGVQE (SEQ ID NO: 1), SYRGTYSTTVTGR (SEQ ID NO: 2), RTPEYYPNAGLI (SEQ ID NO: 3), PDAVAAPY (SEQ ID NO: 4), CSDAEGTAVA (SEQ ID NO: 5), TPVPSLE (SEQ ID NO: 6), LETPTVVPVPS (SEQ ID NO: 7), TFIPGTNK (SEQ ID NO: 8) oder Teilsequenzen davon mit mindestens 4 Aminosäuren zur affinitätschromatographischen Aufreinigung von Antikörpern gegen Lp(a), dadurch gekennzeichnet, daß das Peptid an ein Trägermaterial gebunden wird, an dem dann der Antikörper in bekannter Weise affinitätschromatographisch aufgereinigt werden kann.

2. Verwendung eines Peptids der Sequenzen SEQ ID NO 1-8 oder Teilsequenzen davon mit mindestens 4 Aminosäuren als Immunogen zur Herstellung von Antikörpern gegen Lp(a).

3. Verwendung eines Peptids der Sequenzen SEQ ID NO 1-8 oder Teilsequenzen davon mit mindestens 4 Aminosäuren als Standard in einem immunologischen Test zur quantitativen Bestimmung von Lp(a).

4. Verwendung eines Peptids der Sequenzen SEQ ID NO 1-8 oder Teilsequenzen davon mit mindestens 4 Aminosäuren als Hapten in einem kompetitiven immunologischen Test.

5. Verwendung eines Peptids der Sequenzen SEQ ID NO 1-8 oder Teilsequenzen davon mit mindestens 4 Aminosäuren als Lp(a)-Hapten in einem Agglutinationstest, dadurch gekennzeichnet, daß mindestens ein Peptid der Sequenzen SEQ ID NO 1-8 an einen Träger gebunden wird, so daß sich nach Inkubation des dadurch gebildeten Komplexes mit Antikörpern gegen dieses Hapten ein Agglutinat bildet, dessen Ausbildung in Gegenwart von freiem Lp(a) vermindert wird.

6. Verfahren zur immunologischen Bestimmung von Lp(a) über einen kompetitiven Agglutinationstest durch Inkubation eines trägergebundenen Lp(a)-Haptens mit einem Antikörper gegen dieses Hapten und der zu analysierenden Probe und Messung des dabei auftretenden Meßsignals in Gegenwart und Abwesenheit der zu analysierenden Probe, dadurch gekennzeichnet, daß das trägergebundene Lp(a)-Hapten mindestens ein Peptid der Sequenzen SEQ ID NO 1-8 oder Teilsequenzen davon mit mindestens 4 Aminosäuren enthält.

7. Verfahren zur immunologischen Bestimmung von Lp(a) durch Inkubation eines Marker-gebundenen Lp(a)-Haptens mit einem Antikörper gegen dieses Hapten und der zu analysierenden Probe und Messung des dabei auftretenden Meßsignals in Gegenwart und Abwesenheit der zu analysierenden Probe. dadurch gekennzeichnet. daß das Marker-gebundene Lp(a)-Hapten mindestens ein Peptid der Sequenzen SEQ ID NO 1-8 oder Teilsequenzen davon mit mindestens 4 Aminosäuren enthält.

8. Verfahren gemäß Anspruch 7, dadurch gekennzeichnet, daß es nach dem FPIA-, EMIT- oder CEDIA-Prinzip durchgeführt wird.

9. Verfahren zur affinitätschromatischen Aufreinigung von Antikörpern gegen Lp(a), wobei ein erfindungsgemäßes Peptid der Sequenzen SEQ ID No 1-8 oder Teilsequenzen davon mit mindestens 4 Aminosäuren an ein Trägermaterial gebunden ist, an den der Antikörper dann in an sich bekannter Weise affinitätschromatisch aufgereinigt wird.

10. Verfahren zur Herstellung von Antikörpern gegen Lp(a), die nur eine geringe Reaktivität mit LDL und/oder Plasminogen aufweisen, wobei als Immunogen ein Peptid der Sequenz SEQ ID NO 1-8 oder Teilsequenzen davon mit mindestens 4 Aminosäuren eingesetzt wird.

11. Verwendung von Antikörpern erhältlich durch ein Verfahren nach Anspruch 9 oder 10 bei der immunologischen Bestimmung von Lp(a).

## Claims

1. Use of a peptide having the sequences SEQ ID NO 1 - 8 QAPTEQRPGVQE (SEQ ID NO: 1), SYRGTYSTTVTGR (SEQ ID NO: 2), RTPEYYPNAGLI (SEQ ID NO: 3), PDAVAAPY (SEQ ID NO: 4), CSDAEGTAVA (SEQ ID NO: 5), TPVPSLE (SEQ ID NO: 6), LETPTVVPVPS (SEQ ID NO: 7), TFIPGTNK (SEQ ID NO: 8) or partial sequences thereof with at least 4 amino acids for the purification of antibodies against Lp(a) by means of affinity chromatography, wherein the peptide is bound to a carrier material on which the antibody can then be purified in a known way by means of affinity chromatography.

2. Use of a peptide having the sequences SEQ ID NO 1 - 8 or partial sequences thereof with at least 4 amino acids as an immunogen for producing antibodies against Lp(a).

3. Use of a peptide having the sequences SEQ ID NO 1 - 8 or partial sequences thereof with at least 4 amino acids as a standard in an immunological test for the quantitative determination of Lp(a).

4. Use of a peptide having the sequences SEQ ID NO 1 - 8 or partial sequences thereof with at least 4 amino acids as a hapten in a competitive immunological test.

5. Use of a peptide having the sequences SEQ ID NO 1 - 8 or partial sequences thereof with at least 4 amino acids as an Lp(a) hapten in an agglutination test, wherein at least one peptide having the sequences SEQ ID NO 1 - 8 is bound to a carrier so that after incubation of the complex which is thus formed with antibodies against this hapten an agglutinate forms whose formation is decreased in the presence of free Lp(a).

6. Method for the immunological determination of Lp(a) by a competitive agglutination test by incubating a carrier-bound Lp(a) hapten with an antibody against this hapten and the sample to be analysed and measuring the signal which occurs in this process in the presence and absence of the sample to be analysed, wherein the carrier-bound Lp(a) hapten contains at least one peptide having the sequences SEQ ID NO 1 - 8 or partial sequences thereof with at least 4 amino acids.

7. Method for the immunological determination of Lp(a) by incubating an Lp(a)-hapten bound to a label with an antibody against this hapten and the sample to be analysed and measuring the signal which occurs in this process in the presence and absence of the sample to be analysed, wherein the Lp(a)-hapten bound to the label contains at least one peptide having the sequences SEQ ID NO 1 - 8 or partial sequences thereof with at least 4 amino acids.

8. Method as claimed in claim 7, wherein it is carried out according to the FPIA, EMIT or CEDIA technique.

9. Process for the purification of antibodies against Lp(a) by affinity chromatography in which a peptide according to the invention having the sequences SEQ ID NO 1 - 8 or partial sequences thereof with at least 4 amino acids is bound to a carrier material on which the antibody is then purified in a known manner.

10. Process for the production of antibodies against Lp(a) which have only a low reactivity with LDL and/or plasminogen in which a peptide having the sequences SEQ ID NO 1 - 8 or partial sequences thereof with at least 4 amino acids is used as an immunogen.

11. Use of antibodies obtainable by a process as claimed in claim 9 or 10 for the immunological determination of Lp(a).

## Revendications

1. Utilisation d'un peptide avec les séquences SEQ ID NO : 1 - 8 QAPTEQRPGVQE (SEQ ID NO : 1), SYRGTYSTTVTGR (SEQ ID NO : 2), RTPEYYPNAGLI (SEQ ID NO : 3), PDAVAAPY (SEQ ID NO : 4), CSDAEGTAVA (SEQ ID NO : 5), TPVPSLE (SEQ ID NO : 6), LETPTVVPVPS (SEQ ID NO : 7), TFIPGTNK (SEQ ID NO : 8) ou des séquences partielles de celles-ci d'au moins 4 acides aminés pour l'épuration par chromatographie d'affinité d'anticorps contre Lp(a), caractérisée en ce que le peptide est lié à un matériau de support sur lequel l'anticorps peut ensuite être épuré par chromatographie d'affinité d'une manière connue.

2. Utilisation d'un peptide avec les séquences SEQ ID NO : 1 - 8 ou des séquences partielles de celles-ci d'au moins 4 acides aminés à titre d'agent immunogène pour la préparation d'anticorps contre Lp(a).

3. Utilisation d'un peptide avec les séquences SEQ ID NO : 1 - 8 ou des séquences partielles de celles-ci d'au moins 4 acides aminés à titre de standard dans un test immunologique pour la détermination quantitative de Lp(a).

4. Utilisation d'un peptide avec les séquences SEQ ID NO : 1 - 8 ou des séquences partielles de celles-ci d'au moins 4 acides aminés à titre d'haptène dans un test immunologique compétitif.

5. Utilisation d'un peptide avec les séquences SEQ ID NO : 1 - 8 ou des séquences partielles de celles-ci d'au moins 4 acides aminés à titre d'haptène de Lp(a) dans un test d'agglutination, caractérisée en ce qu'au moins un peptide avec les séquences SEQ ID NO : 1 - 8 est lié à un support de telle manière qu'après incubation du complexe ainsi formé avec anticorps contre cet haptène, il se forme un agglutinat dont la formation est réduite en présence de Lp(a) libre.

6. Procédé de détermination immunologique de Lp(a) par un test d'agglutination compétitif par incubation d'un haptène de Lp(a) lié à un support avec un anticorps contre cet haptène et de l'échantillon à analyser et par mesure du signal de mesure résultant en présence et en absence de l'échantillon à analyser, caractérisé en ce que l'haptène de Lp(a) lié à un support contient au moins un peptide avec les séquences SEQ ID NO : 1 - 8 ou des séquences partielles de celles-ci d'au moins 4 acides aminés.

7. Procédé de détermination immunologique de Lp(a) par incubation d'un haptène de Lp(a) lié à un marqueur avec un anticorps contre cet haptène et de l'échantillon à analyser et par mesure du signal de mesure résultant en présence et en absence de l'échantillon à analyser, caractérisé en ce que l'haptène de Lp(a) lié à un marqueur contient au moins un peptide avec les séquences SEQ ID NO : 1 - 8 ou des séquences partielles de celles-ci d'au moins 4 acides aminés.

8. Procédé selon la revendication 7, caractérisé en ce qu'il est effectué suivant le principe FPIA, EMIT ou CEDIA.

9. Procédé d'épuration par chromatographie d'affinité d'anticorps contre Lp(a), dans lequel un peptide conforme à la présente invention avec les séquences SEQ ID NO : 1 - 8 ou des séquences partielles de celles-ci d'au moins 4 acides aminés est lié à un matériau de support sur lequel l'anticorps est ensuite épuré par chromatographie d'affinité d'une manière connue.

10. Procédé pour la préparation d'anticorps contre Lp(a) qui ne présentent qu'une faible réactivité avec la LDL et/ou le plasminogène, dans lequel on utilise, à titre d'agent immunogène, un peptide avec la séquence SEQ ID NO : 1 - 8 ou des séquences partielles de celles-ci d'au moins 4 acides aminés.

11. Utilisation d'anticorps pouvant être obtenus par un procédé selon la revendication 9 ou 10 dans la détermination immunologique de Lp(a).
